# EUROPEAN PATENT SPECIFICATION

(11) **EP 2 932 240 B1**
(45) Date of publication and mention of the grant of the patent: **23.08.2017**
(21) Application number: 12809707.8
(22) Date of filing: 13.12.2012
(51) Int. Cl.: G01N 21/35, G01N 21/59, G01N 21/85, G01N 33/28

(54) **OPTICAL SENSOR FOR OIL SATURATION**
OPTISCHER SENSOR FÜR ÖLSÄTTIGUNG
CAPTEUR OPTIQUE POUR SATURATION D'HUILE

(43) Date of publication of application: 21.10.2015
(73) Proprietor: Aktiebolaget SKF, 415 50 Göteborg (SE)
(72) Inventor: TATAR, Florin, NL-2622 GE Delft (NL); HERDIER, Romain, B-1180 Uccle (BE); LEWIS, Elfed, Obreiens Bridge Co Clare (IE); DOOLY, Gerard, Annacotty Limerick (IE)
(74) Representative: Tweedlie, Diane Harkness
(86) International application number: PCT/EP2012/075437
(87) International publication number: WO 2014/090315

(56) References cited:
- EP-A1- 2 009 438
- WO-A2-2011/073789
- DE-A1- 3 712 879
- US-A1- 2008 024 761

## Description

### BACKGROUND OF THE INVENTION

### 1. Field of the Invention

The present invention relates to oil condition monitoring and in particular, to a sensor for detecting the saturation level of water in oil or like substances. The invention also relates to a method of monitoring the presence of water in oil and in particular to a method which allows easy auto-calibration of the sensor in-situ.

### 2. Description of the Related Art

Optical sensors have been used for oil condition monitoring for determining the presence of debris or otherwise monitor deterioration of a lubricant. Such devices may operate by shining light through a small gap and analysing the transmitted light with a suitable optical sensor. Alternative sensors may make use of scattering of light and may operate over different frequencies including outside of the visible range. Oil condition monitoring may be significant in providing feedback in advance of likely failure of a lubricant system. Action may be taken to perform maintenance or otherwise renew the lubricant.

Water in oil is of considerable concern to many mechanical systems. Minimal amounts of water may be absorbed by the oil during use, either from the atmosphere or by direct ingress of water into the system. As long as this water is in the absorbed state and the oil is unsaturated, the concern is minimal. Nevertheless, as the concentration of water approaches the saturation level, emulsified and free water may occur, which can be highly detrimental, especially if exposure is prolonged. In bearings, the incompressibility of water relative to the oil can result in disruption of the oil film leading to excessive wear. Just one percent water in oil can reduce the life expectancy of a bearing by as much as 90 percent. For ball or rolling element bearings, the localized pressure generated can cause spontaneous vaporization of the water, leading to erosive wear such as micropitting. The saturation level of water in oil may vary widely according to temperature and the type of oil and can range from 10 ppm to even 10000 ppm. Existing sensors capable of measuring the presence of water (free and dissolved) include capacitive sensors and Karl Fischer titration sensors. Both of these methods require considerable time for the sensor to reach equilibrium and are not ideal for rapidly changing conditions. Spectral analysis using Fourier Transform Infrared Spectroscopy (FTIR) has been used but is a relatively complex and costly procedure requiring calibration of the sensor relative to the spectrum produced with fresh oil.

### BRIEF SUMMARY OF THE INVENTION

According to the invention there is provided a method of in-situ detecting the saturation level of water in oil in a mechanical system, comprising: providing an optical sensor having a gap for transmission of light between an emitter and receiver through a sample of the oil; passing light through the oil from the emitter to the receiver; detecting at the receiver, the light transmitted from the emitter and producing a light signal representative of the light intensity detected; analysing the light signal to determine an amount of fluctuation of the light intensity in time; monitoring the amount of fluctuation of the light signal to identify a step change attributable to the formation of bubbles of free water and generating a saturation signal indicative of saturation if the identified step change exceeds a predetermined value representing saturation of the oil. According to the invention, it has been observed that a significant change in signal characteristic is to be observed at the point at which free water appears in the oil. Below the saturation level, the light signal as received by the receiver is relatively stable and only steadily decreases in intensity with increasing absorbed water content. As the amount of water approaches saturation, the light signal becomes highly unstable and may appear noisy. Without wishing to be bound by theory, it is believed that bubbles of free water are formed within the oil in a manner similar to cavitation or boiling of a liquid. As these bubbles pass the sensor, they disturb the signal, effectively leading to greater absorption of the light and a lower light signal. A significant advantage of the above effect is that a sensor can be easily calibrated in-situ to the saturation level, without requiring knowledge of either the oil or sensor characteristics. Additionally, the sensor can provide real-time results with negligible delay in identifying the presence of free water in the oil.

Determination of the onset of free water and the step change in fluctuation of the signal can be realised in many ways as will be evident to the skilled person. This may be determined visually and manually on reviewing a data stream record of the light signal. Alternatively and preferably, the method may be carried out by a signal processor using an appropriate algorithm. In one embodiment of the invention, the amount of fluctuation may be determined by measuring a maximum peak to peak variation of the light signal within a sampling period. The sampling period may be chosen depending on various factors, including the sampling rate at which measurements of the light signal are taken and also based on physical factors such as the flow rate of the oil being monitored. It will be understood that the sampling period will include at least two samples, preferably at least four samples and more preferably at least 10 samples.

In a preferred method, the sampling period is between 1 second and 10 seconds, preferably between 2 second and 5 seconds. A sample rate of between 1 Hz and 10 Hz may be used, preferably around 2Hz, again depending on the flow rate. In general, for a higher flow rate of the oil, a higher sample rate may be required for the same sensitivity.

According to one method of analysis, saturation may be identified when a ratio of the light signal fluctuation in a second sampling period to the light signal fluctuation in a first sampling period exceeds a predetermined value. The predetermined value may be determined according to the nature of the oil and other physical factors. In general, with the onset of free water the signal fluctuation may increase ten-fold or more and a predetermined value of 5 may be sufficient to provide reliable indication while avoiding false alarms. In other circumstances, a predetermined value of 2 may provide more sensitive response and predetermined values of below 2 may be applicable, in particular where signal smoothing has previously been applied.

According to a further aspect of the invention, the method may further comprise determining the time that the oil remains above its saturation level. Once free water is detected in the oil, a timer may register the time elapsed until the danger of free water has receded. This point may be determined by evaluating a number of successive sampling periods and determining that water is absent once absence of saturation signal has been determined for all of these periods. Alternatively, once the absolute value of the light signal corresponding to the saturation level has been determined, the absence of free water may be indicated once the absolute value of the light signal returns to a value distant from the saturation level.

According to an alternative method, the exposure of the system to free water may be determined by integrating the saturation signal with respect to time. Based on the flow rate of the oil through the sensor, integration of the signal may allow an approximate determination of the total amount of free water in the system. This may be used to provide further alarms and initiate appropriate actions in the event that a given exposure is exceeded.

According to the invention, it has been found that the method is particularly effective for infra red light in the range from 850 nm to 1750 nm. Near infra red light shows good absorption properties for water while being only slightly affected by other contaminants in the oil. The light may be analysed over a broad spectrum e.g. using a spectroanalyser. Most preferably, the light is analysed in regions of the spectrum where water absorption peaks are present. For most oils, such absorption peaks may be identified at around 1250 nm and around 1400 nm and analysis in one or both of these specific regions is preferred.

As described, a significant advantage of the present invention is that the sensor need not be pre-calibrated and may be calibrated in-situ to the saturation level. In the event that greater accuracy is required in the region of absorbed water, the method may comprise calibrating the sensor for a sample of oil having a water content below the saturation level and subsequently determining a linear relation between the light signal and the water content when saturation of the oil is detected. Such a simple calibration may be achieved in a laboratory by calibrating the sensor against a Karl Fischer titration result. Alternatively, the sensor may be calibrated in the field by taking an oil sample for off-line analysis. Once calibrated, the sensor may be accurately used to also identify emulsified water in oil before the advent of free water.

The invention also relates to a system for detecting the saturation level of water in oil, the system comprising: an optical sensor having an emitter and a receiver, separated by a gap for transmission of light therebetween through a sample of the oil, the receiver being arranged to detect light transmitted through the oil from the emitter and produce a light signal representative of the light intensity detected; a processor arranged to analyse the light signal produced by the receiver and determine an amount of fluctuation of the light signal in time; and a memory for storing data relating to a preset value for an expected fluctuation of the light signal attributable to the formation of bubbles of free water and indicative of full saturation of the oil. The system may be implemented to operate according to the method as described above.

According to the invention, the optical sensor may have a gap of less than 2 mm, preferably less than 1 mm and most preferably around 0.5 mm. It has been found that increase light sensitivity may be achieved with a smaller gap. Nevertheless, a gap of around 0.5 mm is considered optimal, as it allows more than sufficient light transmission for most oils and is not so narrow that clogging with debris could occur. It will be understood that for relatively more viscous oils a larger gap might be considered than for lighter oils.

In one embodiment, the emitter comprises a broad spectrum lamp and the receiver comprises a spectroanalyser capable of analysing the light signal across the range, preferably at least across the relevant wavelengths as discussed above.

In an alternative embodiment, the emitter comprises one or more light emitting diodes (LEDs) and the receiver comprises one or more photodiodes, operating at the corresponding frequency. The LEDs may thus be coupled to a respective photodiode across the gap.

The processor may be any appropriate processing device such as a computer or dedicated microprocessor. In addition to other control tasks, the processor is arranged to determine when the fluctuation of the light signal exceeds the preset value. In particular the processor may carry out signal analysis, sampling and filtering as described above.

The skilled person will understand that the sensor of the present invention may be implemented in a number of different situations where water in oil is to be recorded. Preferably, the optical sensor is located in an oil supply line to a mechanical system. The mechanical system may be a motor, a gear, a bearing, a journal, a cam or a complex system comprising one or more of the above.

### BRIEF DESCRIPTION OF THE DRAWINGS

The features and advantages of the invention will be appreciated upon reference to the following drawings of a number of exemplary embodiments, in which:
Figure 1 shows a schematic view of a system according to the present invention;
Figure 2 shows a graph of light intensity signal detected by the system of Figure 1 over the range of wavelengths from 850nm to 1700 nm;
Figure 3 shows light intensity measurements for increasing water content measured in the system of Figure 1 for the wavelength of 1108 nm; and
Figure 4 shows relative humidity measurements for the system of Figure 1 in parallel with commercial sensors.

### DESCRIPTION OF ILLUSTRATIVE EMBODIMENTS

### Example 1

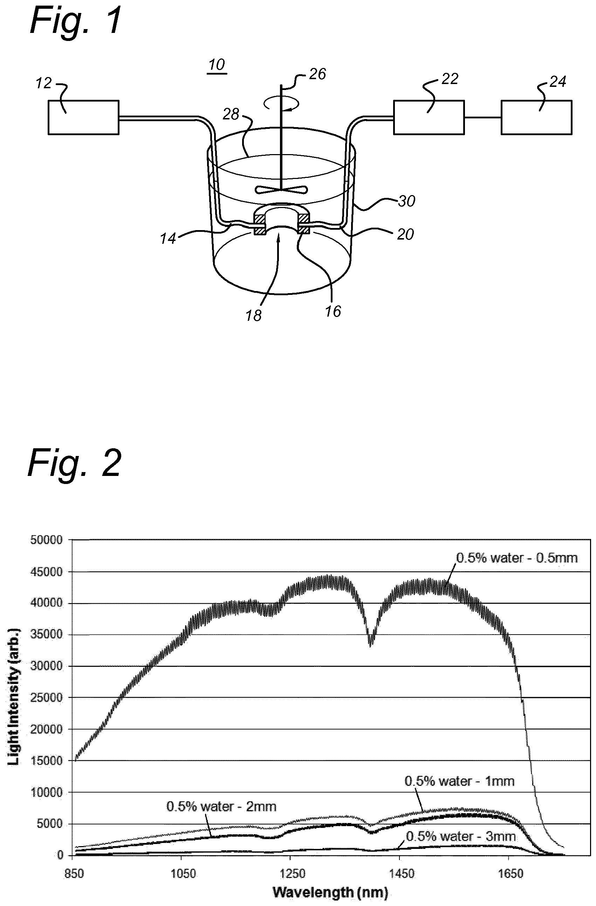

Figure 1 shows a schematic view of an experimental set up of a system 10 according to the present invention, comprising a deuterium/halogen lamp source 12 coupled to an optical fibre emitter 14. The optical fibre emitter 14 is coupled to a detection cell 16 comprising a gap 18. Facing the optical fibre emitter 14 across the gap 18 is an optical fibre receiver 20 coupled to a spectroanalyser 22. The spectroanalyser 22 is in turn connected to a personal computer 24. Both the optical fibre emitter 14 and optical fibre receiver 20 comprised optical fibres of 400 mm length. A stirrer 26 is provided to circulate the oil 28 contained in beaker 30.

In use, the detection cell 16 is immersed in the oil 28 or other lubricant to be monitored. The location of the cell 16 in the lubricant system will depend on the specific situation but it may preferably be provided in a delivery line, leading from a sump to the mechanism to be lubricated.

In an initial test, the effects on the system 10 with regards to the width of the gap 18 within the detection cell 16 were investigated. For this a number of different detection cells 16 were constructed having gap widths of 3mm, 2mm, 1mm and 0.5mm respectively. For this example, the system 10 of Figure 1 was applied to a beaker 30 of mineral oil (Total Cirkan C100^{™}), placed in a controlled humidity/temperature chamber. Figure 2 illustrates the resulting light intensity signal detected by the spectroanalyser 22 over the range of wavelengths from 850nm to 1700 nm, the light intensity being given on an arbitrary scale from 0 to 50 000. As can be seen, the light intensity measurements for the 0.5 mm gap are considerably higher than for the larger gaps and also offer a significantly greater signal to noise ratio for subsequent analysis.

### Example 2

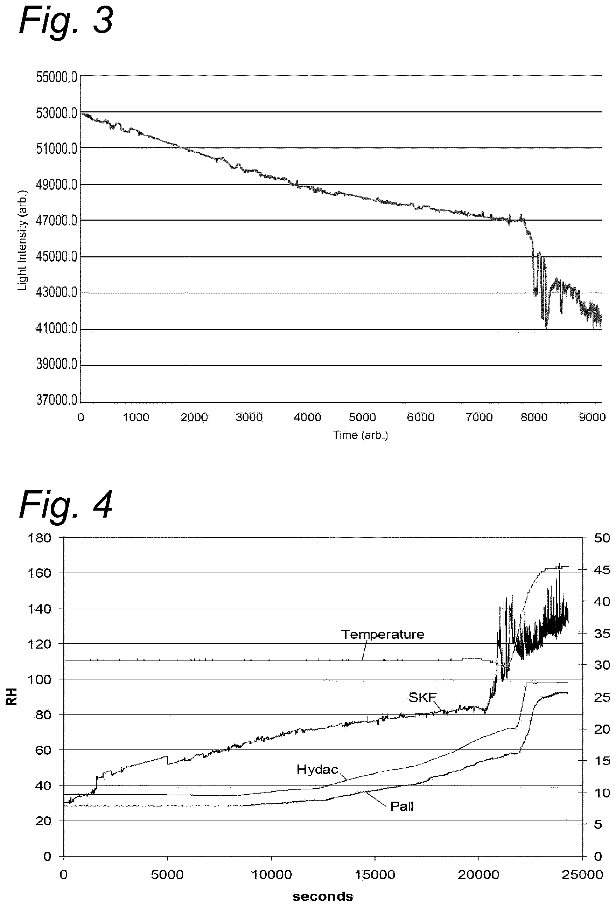

In a subsequent test, the same system 10 was used as in Example 1, whereby the 0.5 mm gap detection cell 16 was employed. The full spectrum was recorded over a number of hours, during which the concentration of the water was steadily increased until the saturation level was reached (100% saturated). After this the water concentration was further increased to about 0.4% water. The light intensity measurements for the full experimental test are shown in Figure 3 for the wavelength of 1108 nm. The result shows a strong and stable response to the ever increasing levels of water content. As the sample reaches saturation, the sensor records significant fluctuation in the signal. The sampling rate was 2 Hz. The skilled person will recognize that digital filtering such as a moving point average could be used to further smooth the signal.

### Example 3

The measurements as performed in Example 2 were repeated in a controlled climate chamber. By adjusting the humidity steadily, it could be assumed that the oil would be in equilibrium with the air in the chamber and have the same relative humidity. The measurements were performed in parallel with commercial (Hydac and Pal) capacitance sensors which absorb or desorb moisture, to achieve equilibrium with the surrounding fluid. The results are displayed in Figure 4 in which the relative humidity value is based on the reading for the controlled climate within the chamber. After 3 hours of recording, the Hydac and Pal sensors did not show significant variation of the saturation measured values (30% and respectively 35%), while the detection cell 16 indicated 70% saturation. At this point, visual observation of the oil showed that it had changed from very transparent to non-transparent. The atmospheric humidity of the chamber was 100% humidity. Following further measurements, when the oil reached a milky observed colour indicating the emulsified state, the detection cell 10 registered absolute humidity of 100%. At this point, the Pal and Hydac sensors registered within the 50-70% range respectively. The results as plotted in Figure 4 appear to show a delay of 1 to 3 hours for these sensors with respect to the actual values. It is also noted that beyond saturation in the free water region, the detection cell 16 continues to show variation and allows calibration and measurement in this region. The Pal and Hydac sensors reach a plateau at saturation and do not register the free water content.

The above tests were repeated for polyglycol with similar results. Based on the above it appears that the sensor portrays a significant level of sensitivity (20ppm), a wide range of detection (20ppm - 10000ppm) and fast response and recovery times.

Thus, the invention has been described by reference to the embodiment discussed above. It will be recognized that this embodiment is susceptible to various modifications and alternative forms well known to those of skill in the art without departing from the spirit and scope of the invention. In particular, for implementation in a mechanical system, the detection cell may be located in an oil supply line whereby a portion of the oil supply passes through the gap. Furthermore, the personal computer may be replaced by a dedicated microprocessor which may be located in-situ or remotely. Accordingly, although specific embodiments have been described, these are examples only and are not limiting upon the scope of the invention.

## Claims

1. A method of in-situ detecting the saturation level of water in oil in a mechanical system, comprising:
providing an optical sensor having a gap for transmission of light between an emitter and receiver through a sample of the oil;
passing light through the oil from the emitter to the receiver;
detecting at the receiver, the light transmitted from the emitter and producing a light signal representative of the detected light intensity;
**characterized by**
analysing the light signal to determine an amount of fluctuation of the light intensity in time; and
monitoring the amount of fluctuation of the light signal to identify a step change attributable to the formation of bubbles of free water and generating a saturation signal indicative of saturation if the identified step change exceeds a predetermined value representing saturation of the oil.

2. The method according to claim 1, wherein determining an amount of fluctuation comprises measuring a peak to peak variation of the light signal within a sampling period.

3. The method according to claim 2, wherein the sampling period is between 0.5 seconds and 10 seconds, preferably between 1 second and 3 seconds.

4. The method according to claim 2 or claim 3, wherein saturation is identified when a ratio of the light signal fluctuation in a second sampling period to the light signal fluctuation in a first sampling period exceeds a predetermined value.

5. The method according to any preceding claim, further comprising determining the time that the oil remains above its saturation level.

6. The method according to claim 5, further comprising integrating the saturation signal with respect to time.

7. The method according to any preceding claim, wherein the light comprises infra red light in the range 850 nm to 1750 nm.

8. The method according to claim 7, wherein the light is analysed in the regions of 1250 nm and/or 1400 nm.

9. The method according to any preceding claim, further comprising calibrating the sensor for a sample of oil having a water content below the saturation level and subsequently determining a linear relation between the light signal and the water content when saturation of the oil is detected.

10. A system for detecting the saturation level of water in oil, the system comprising:
an optical sensor having an emitter and a receiver, separated by a gap for transmission of light therebetween through a sample of the oil, the receiver being arranged to detect light transmitted through the oil from the emitter and produce a light signal representative of the detected light intensity;
a processor arranged to analyse the light signal produced by the receiver and determine an amount of fluctuation of the light signal in time; and
a memory for storing data relating to a preset value for an expected fluctuation of the light signal attributable to the formation of bubbles of free water and indicative of full saturation of the oil,
whereby the processor is further arranged to determine when the fluctuation of the light signal exceeds the preset value.

11. The system of claim 10, wherein the optical sensor has a gap of less than 2 mm, preferably less than 1 mm and most preferably around 0.5 mm.

12. The system of any of claims 10 or 11, wherein the emitter comprises a broad spectrum lamp and the receiver comprises a spectroanalyser.

13. The system of any of claims 10 or 11, wherein the emitter comprises one or more light emitting diodes and the receiver comprises one or more photodiodes, operating at the corresponding frequency.

14. The system according to any of claims 10 to 13, wherein the optical sensor is located in an oil supply line to a mechanical system.

## Patentansprüche

1. Verfahren zum Detektieren in situ des Sättigungspegels von Wasser in Öl in einem mechanischen System, das umfasst:
Bereitstellen eines optischen Sensors, der einen Spalt zur Übertragung von Licht durch eine Probe des Öls hindurch zwischen einem Emitter und einem Empfänger aufweist;
Durchlassen von Licht durch das Öl von dem Emitter zu dem Empfänger;
Detektieren des Lichts, das von dem Emitter übertragen wird, an dem Empfänger und Produzieren eines Lichtsignals, das für die detektierte Lichtintensität charakteristisch ist;
**gekennzeichnet durch**
Analysieren des Lichtsignals zum Bestimmen eines Betrags an Schwankung der Lichtintensität im Lauf der Zeit; und
Überwachen des Betrags an Schwankung des Lichtsignals zum Identifizieren einer sprunghaften Veränderung, die auf die Bildung von Blasen von freiem Wasser zurückzuführen ist;
und Erzeugen eines Sättigungssignals, das die Sättigung anzeigt, wenn die identifizierte sprunghafte Veränderung einen vorbestimmten Wert übersteigt, der die Sättigung des Öls darstellt.

2. Verfahren nach Anspruch 1, wobei das Bestimmen eines Betrags an Schwankung das Messen einer Spitze-zu-Spitze-Schwankung des Lichtsignals in einer Abtastperiode umfasst.

3. Verfahren nach Anspruch 2, wobei die Abtastperiode zwischen 0,5 Sekunden und 10 Sekunden, vorzugsweise zwischen 1 Sekunde und 3 Sekunden liegt.

4. Verfahren nach Anspruch 2 oder Anspruch 3, wobei die Sättigung identifiziert wird, wenn ein Verhältnis der Lichtsignalschwankung in einer zweiten Abtastperiode zu der Lichtsignalschwankung in einer ersten Abtastperiode einen vorbestimmten Wert übersteigt.

5. Verfahren nach einem der vorhergehenden Ansprüche, das ferner das Bestimmen der Zeit, zu der das Öl über seinem Sättigungspegel bleibt, umfasst.

6. Verfahren nach Anspruch 5, das ferner das Integrieren des Sättigungssignals relativ zu der Zeit umfasst.

7. Verfahren nach einem der vorhergehenden Ansprüche, wobei das Licht Infrarotlicht im Bereich von 850 nm bis 1750 nm umfasst.

8. Verfahren nach Anspruch 7, wobei das Licht in den Regionen von 1250 nm und/oder 1400 nm analysiert wird.

9. Verfahren nach einem der vorhergehenden Ansprüche, das ferner das Kalibrieren des Sensors für eine Probe von Öl mit einem Wassergehalt unterhalb des Sättigungspegels und das anschließende Bestimmen einer linearen Beziehung zwischen dem Lichtsignal und dem Wassergehalt, wenn eine Sättigung des Öls detektiert wird, umfasst.

10. System zum Detektieren des Sättigungspegels von Wasser in Öl, wobei das System umfasst:
einen optischen Sensor mit einem Emitter und einem Empfänger, die durch einen Spalt zum Übertragen von Licht zwischen diesen durch eine Probe des Öls hindurch voneinander getrennt sind, wobei der Empfänger so ausgelegt ist, dass er Licht, das von dem Emitter durch das Öl übertragen wird, detektiert und ein Lichtsignal produziert, das für die detektierte Lichtintensität charakteristisch ist;
einen Prozessor, der so ausgelegt ist, dass er das Lichtsignal, das von dem Empfänger produziert wird, analysiert und einen Betrag an Schwankung des Lichtsignals im Lauf der Zeit bestimmt; und
einen Speicher zum Speichern von Daten, die sich auf einen vorgegebenen Wert für eine erwartete Schwankung des Lichtsignals, welche auf die Bildung von Blasen von freiem Wasser zurückzuführen ist, beziehen und eine vollständige Sättigung des Öls anzeigen,
wobei der Prozessor ferner so ausgelegt ist, dass er bestimmt, wann die Schwankung des Lichtsignals den vorgegebenen Wert übersteigt.

11. System nach Anspruch 10, wobei der optische Sensor einen Spalt von weniger als 2 mm, vorzugsweise weniger als 1 mm und am stärksten bevorzugt von ungefähr 0,5 mm aufweist.

12. System nach einem der Ansprüche 10 oder 11, wobei der Emitter eine Breitspektrumlampe umfasst und der Empfänger einen Spektrumanalysator umfasst.

13. System nach einem der Ansprüche 10 oder 11, wobei der Emitter eine oder mehrere Leuchtdioden umfasst und der Empfänger eine oder mehrere Fotodioden umfasst, die bei der entsprechenden Frequenz arbeiten.

14. System nach einem der Ansprüche 10 bis 13, wobei sich der optische Sensor in einer Ölzuführleitung zu einem mechanischen System befindet.

## Revendications

1. Procédé de détection in situ du niveau de saturation en eau d'une huile dans un système mécanique, comprenant les étapes suivantes :
se procurer un capteur optique ayant un espace pour la transmission de lumière entre un émetteur et un récepteur à travers un échantillon de l'huile ;
faire passer de la lumière à travers l'huile depuis l'émetteur jusqu'au récepteur ;
détecter au niveau du récepteur la lumière transmise depuis l'émetteur et produire un signal lumineux représentatif de l'intensité lumineuse détectée ;
**caractérisé par** les étapes suivantes :
analyser le signal lumineux pour déterminer une quantité de fluctuation de l'intensité lumineuse dans le temps ; et
surveiller la quantité de fluctuation du signal lumineux pour identifier un changement brusque imputable à la formation de bulles d'eau libre et générer un signal de saturation révélateur d'une saturation si le changement brusque identifié dépasse une valeur prédéterminée représentant une saturation de l'huile.

2. Procédé selon la revendication 1, dans lequel la détermination d'une quantité de fluctuation comprend la mesure d'une variation de pic à pic du signal lumineux à l'intérieur d'une période d'échantillonnage.

3. Procédé selon la revendication 2, dans lequel la période d'échantillonnage se situe entre 0,5 seconde et 10 secondes, de préférence entre 1 seconde et 3 secondes.

4. Procédé selon la revendication 2 ou la revendication 3, dans lequel une saturation est identifiée quand un rapport entre la fluctuation du signal lumineux dans une deuxième période d'échantillonnage et la fluctuation du signal lumineux dans une première période d'échantillonnage dépasse une valeur prédéterminée.

5. Procédé selon une quelconque revendication précédente, comprenant en outre la détermination du temps pendant lequel l'huile reste au-dessus de son niveau de saturation.

6. Procédé selon la revendication 5, comprenant en outre l'intégration du signal de saturation par rapport au temps.

7. Procédé selon une quelconque revendication précédente, dans lequel la lumière comprend une lumière infrarouge dans la gamme de 850 nm à 1750 nm.

8. Procédé selon la revendication 7, dans lequel la lumière est analysée dans les régions de 1250 nm et/ou 1400 nm.

9. Procédé selon une quelconque revendication précédente, comprenant en outre l'étalonnage du capteur pour un échantillon d'huile ayant une teneur en eau inférieure au niveau de saturation et la détermination consécutive d'une relation linéaire entre le signal lumineux et la teneur en eau quand une saturation de l'huile est détectée.

10. Système de détection du niveau de saturation en eau d'une huile, le système comprenant :
un capteur optique ayant un émetteur et un récepteur, séparés par un espace pour la transmission de lumière entre eux à travers un échantillon d'huile, le récepteur étant agencé pour détecter la lumière transmise à travers l'huile depuis l'émetteur et produire un signal lumineux représentatif de l'intensité lumineuse détectée ;
un processeur agencé pour analyser le signal lumineux produit par le récepteur et déterminer une quantité de fluctuation du signal lumineux dans le temps ; et
une mémoire destinée à stocker des données relatives à une valeur prédéfinie pour une fluctuation prévue du signal lumineux imputable à la formation de bulles d'eau libre et révélatrice d'une saturation complète de l'huile,
le processeur étant également agencé pour déterminer le moment où la fluctuation du signal lumineux dépasse la valeur prédéfinie.

11. Système de la revendication 10, dans lequel le capteur optique a un espace inférieur à 2 mm, de préférence inférieur à 1 mm et idéalement d'environ 0,5 mm.

12. Système de l'une quelconque des revendications 10 ou 11, dans lequel l'émetteur comprend une lampe à large spectre et le récepteur comprend un analyseur spectral.

13. Système de l'une quelconque des revendications 10 ou 11, dans lequel l'émetteur comprend une ou plusieurs diodes électroluminescentes et le récepteur comprend une ou plusieurs photodiodes, fonctionnant à la fréquence correspondante.

14. Système selon quelconque des revendications 10 à 13, dans lequel le capteur optique est situé dans une conduite d'alimentation en huile d'un système mécanique.
